# EUROPEAN PATENT SPECIFICATION

(11) **EP 1 195 099 B1**
(45) Date of publication and mention of the grant of the patent: **15.03.2017**
(21) Application number: 01402155.4
(22) Date of filing: 10.08.2001
(51) Int. Cl.: A61K 8/66, A61Q 11/00, A61Q 15/00, C11D 3/50, A23G 3/36, A23G 4/06, A23G 4/12, A61Q 5/02, A61L 11/00, C11D 3/00, C11D 3/20, C11D 3/38, A61K 8/34, A61K 8/97, A61L 9/01, C11D 3/382, C11D 3/386

(54) **Deodorant composition and its application**
Deodorant-Zubereitung und deren Verwendung
Composition désodorisante et son application

(30) Priority: 11.08.2000 US 637076
(43) Date of publication of application: 10.04.2002
(73) Proprietor: Takasago International Corporation, Tokyo 144-8721 (JP)
(72) Inventor: Nakatsu, Tetsuo, Chappaqua, N.Y. 10514 (US); Hiramoto, Tadahiro, Hiratsuka-shi, Kanagawa (JP); Saiki, Kenji, Hiratsuka-shi, Kanagawa (JP); Mishima, Yasutaka, Hiratsuka-shi, Kanagawa (JP)
(74) Representative: Uchida, Kenji

(56) References cited:
- EP-A- 0 979 612
- US-A- 5 880 076
- US-A- 6 074 631

## Description

The present invention relates to a deodorizing composition comprising an enzyme-and-substrate mixture (hereinafter referred to as "ESM") which includes an oxidation substrate and an oxidative enzyme capable of oxidizing this oxidation substrate, and one or several types of flavors or fragrances (hereinafter referred to as "FOF"). More specifically, the present invention relates to a deodorizing composition including a phenolic compound as an oxidation substrate; an enzyme capable of oxidizing the phenolic compounds into the compounds having a quinone structure; and FOF.

There are a number of deodorant products presently available that are used to get rid of various malodors in the environments. The main components of such malodors include for example; common nitrogen containing compounds such as ammonia, urea, indole, skatole and amines; sulfur containing compounds such as methyl mercaptan, hydrogen sulfide and dimethyl sulfide; and lower fatty acids such as butyric acid. Of particular concern here are odors associated with halitosis (bad breath), body odor and bodily excretions including feces and urine. These odors are usually associated with production of volatile sulfur compounds such as methyl mercaptan and dimethyl sulfide and nitrogen containing compounds such as amines, ammonia and urea. Specifically, ammonia and urea are a common source of urine malodor. The volatile sulfide compounds, produced by mouth bacteria through food decomposition, are a common source of halitosis (bad breath). In particular, hydrogen sulfide and methyl mercaptan are considered to be a main cause of bad mouth odor. These sources can cause detectable malodors at very low concentrations. The minimum level detectable for human olfactory senses may be defined as a threshold concentration, below which the odor cannot be detected. In particular, sulfide compounds have a very low level of odor threshold concentrations.

Typically, flavors or fragrances can be used in order to shield or mask environmental bad odors. In other words, when some types of odor cannot be inactivated by chemical or physical means, they may be masked by using a high concentration of FOF. However, when these odors are very strong, they cannot be always shielded completely, even if a very high concentration or density of FOF is applied. Furthermore, the bad odors may even be enhanced by some types of perfume.

EP 0 979 612 A1 discloses a method for reducing or eliminating the odour of animal excreta that comprises feeding the animal with an edible material containing extracts selected amongst the extracts of rosemary, sunflower seeds, fresh coffee beans, grape pericarp, grape seeds, apple, carrot leaves and an enzyme capable of oxidizing phenolic compounds.

Also, US 6 074 631 A discloses a method of treating an object with malodour caused by a short fatty acid, comprising treating the object with a combination of one or more oxidoreductase and a mediator.

Further, US 5 804 170 A discloses a composition comprising a phenolic compound and an enzyme capable of oxidizing the phenolic compound wherein the composition has a deodorizing effect.

The present inventors have previously discovered that the deodorizing effect of certain synthetic phenolic compounds is enhanced by combining the phenolic compound with a polyphenol oxidase (e.g. U.S. Patent No. 5,804,170). In addition, certain plant extracts containing phenolic compounds show the same enhanced effect when used in conjunction with an enzyme capable of oxidizing the phenolic compounds (see Japanese Laid-Open Patent No. 10-212221).

In spite of this enhanced effect of the above combined compositions, it was found that the deodorant products themselves still retained a weak remaining odor.

Moreover, none of numerous deodorant compositions available are capable of completely eliminating or masking malodors.

An object of the present invention is therefore to provide a deodorant composition that has a good deodorant or deodorizing effect, and/or shields bad odors, and/or supplies a pleasant flavors. The deodorizing composition of the invention can thus eliminate, or mask or shield, the last remaining odor, which could not be suppressed by the known deodorizing compositions.

Another object of the invention is to provide a deodorizing composition comprising at least one phenolic compound, at least one enzyme capable of oxidizing this phenolic compound and at least one type of FOF.

A further object of the invention is to provide a method of removing or masking malodors, comprising the steps of preparing a deodorizing composition or product comprising the deodorizing composition and an appropriate carrier according to the invention, and of applying it to malodorous substances, or a human or mammals.

To this end, there is provided a deodorant composition comprising at least one phenolic compound and at least one enzyme capable of oxidizing the at least one phenolic compound into a compound having a quinone structure, characterized in that
said deodorant composition further comprises at least one of flavor and fragrance that comprises at least one compound selected from the group consisting of one or several volatile compounds having a molecular weight lower than 500 Dalton; an essential oil; and plant, vegetable or fruit extracts, said deodorant composition comprising said at least one of flavor and fragrance in an amount sufficient to remove the last remaining trace of odours which cannot be inactivated by said at least one phenolic compound and at least one enzyme;
wherein said at least one of flavor and fragrance is citrus-type fragrance, mint-type fragrance, musk- and wood-type fragrance, fruit- and floral-type fragrance, greenery- and floral-type fragrance, peppermint-type flavour, mint-type flavour, lemon-type flavour or perilla-type flavor
Preferably, the amount of said at least one of flavour and fragrance compound to be mixed is at least 0.1% by weight, with respect to the amount of said deodorant composition.

Preferably still, the amount of said at least one of flavour and fragrance compound to be mixed is at least 1% by weight, with respect to the amount of said deodorant composition.

Preferably yet, the one or several volatile compounds having a molecular weight lower than 500 Dalton contain no sulfur atom nor nitrogen atom.

Suitably, when the at least one of flavor and fragrance comprises several compounds and at least one of said several compounds includes a sulfur atom or nitrogen atom, said sulfur- or nitrogen-including compound does not constitute an essential perfumery component in said at least one of fragrance and flavor.

Suitably still, the enzyme comprises at least one enzyme selected from the group consisting of polyphenol oxidase, cathecol oxidase, monophenol oxidase, peroxidase and a plant extract containing an oxidase.

Suitably yet, the polyphenol oxidase comprises a least one enzyme selected from the group consisting of laccase, urushiol oxidase and phenolase.

Typically, the monophenol oxydase comprises tyrosinase.

The invention also relates to a product or article comprising the deodorant composition containing the phenolic compound(s), the enzyme(s), and the flavour and/or fragrance definedabove, as a combined preparation for simultaneous, separate or sequential use.

Preferably, the product or article comprises a detergent, an antiperspirant, a shampoo, a mouth rinse, toothpaste, tablet, candy, a chewing gum, or a food.

The invention further relates to a spray product containing the deodorant composition defined above.

The invention also concerns a method of removing and/or masking malodors comprising the steps of:
mixing at least one phenolic compound, at least one enzyme capable of oxidizing the phenolic compound(s) and at least one of fragrance and flavour, whereby there is provided a deodorant composition as defined above and
applying the deodorant composition to a malodorous object, human or mammal.

Preferably, the mixing step comprises an appropriate carrier, whereby there is provided a carrier-bearing composition including a deodorant composition defined above.

Preferably still, the mixing step comprises an appropriate carrier, whereby there is provided a deodorant product or article defined above.

The present invention is based on the observations that a certain number of FOF substances can react synergistically with a deodorizing agent, and completely eliminate the effects of bad odor.

The present invention relates therefore to a deodorant composition including an enzyme substrate mixture (ESM) which comprises a given substrate and an enzyme capable of oxidizing the substrate, and a FOF substance for masking bad odors.

The quality of fragrance or flavor is judged on the basis of "freshness" (or refreshing feeling), "cleanliness" (or cleanness, or clean feeling) and "pleasant feeling" (or agreeable feeling or "pleasurableness") according to a standardized evaluation method as explained in the present invention. The flavor or fragrance of the invention preferably meets the satisfaction criteria for "freshness", "cleanliness" and "pleasant feeling" mentioned above. The satisfaction criteria are expressed in terms of scores ranging from 0 to 100 points. Typically, the scores exceeding 50 points signify a satisfactory result.

The above, and the other objects, features and advantages of the present invention will be made apparent from the following description of the preferred embodiments, given as non-limiting examples.

The substrates used for the invention are phenolic compounds, preferably polyphenol-type compounds. The phenolic compounds may be natural or synthetic products. The natural phenolic compounds may be extracted from one or several phenol-rich plant(s). For instance, they may be extracted from one or several plants including rosemary, sunflower seeds, grape seeds and pericarp, apple, carrot leaves, bananas, strawberries, apricots, peaches, plums, citrus fruits, pineapples, pears, persimmons (kakis), cherries, papayas, mangos, avocados, melons, loquats, figs, kiwis, prunes, blueberries, black berries, raspberries, cranberries, coffee beans, cacao beans, grapefruit seeds, pecan nuts, cashew nuts, chestnuts, coconuts, peanuts, walnuts, green tea, black tea, oolong tea, tobaccos, perilla leaves, garden thyme, sages, lavenders, spearmints, peppermints, blessed thistle, hyssops, basil, marigolds, dandelions, artichokes, German chamomile, agrimony, licorice, anise, yarrow, eucalyptus, warmwood, a balm, angelica, fenugreek, paprika, fennel, anise seeds, capsicum pepper, cardamom, celery seeds, coriander seeds, cumin seeds, fennel seeds, ginger, horseradish, marjoram, oregano, mustard, parsley, pepper, savory, tarragon, sesame, turmeric, wasabi, clove and dill seeds. These phenolic compounds may be used alone or as a mixture.

The extracts may be obtained by any method known in the art. Specifically, an extract may be obtained by mixing a plant source with a solvent followed by elimination of the solvent, if necessary. Examples of suitable solvents include water, alcohol, organic solvents and mixtures thereof. Preferably, the plant source will be pretreated by drying and cutting the plant into small pieces prior to the extraction process.

Preferably the plant sources will include those rich in phenolic compounds. The choice of the extraction solvent depends on the intended use of the deodorant composition. However, it is preferable to use a solvent that is safe for use in food products such as hot water, water containing ethanol and water containing propylene glycol.

The obtained extracts may be further subjected to a resin adsorption process, so that phenolic compounds can be obtained as a concentrate.

The substrate may also be a synthetic phenolic compound having one or more phenolic hydroxyl groups. The term "phenolic hydroxyl group" used herein includes a hydroxyl group directly bonded to an aromatic ring such as the benzene ring. The aromatic ring may be any of benzene, pyridine, thiophene, naphthalene, biphenyl, and other aromatic rings having a structure that can be converted into ketones by oxidation of hydroxyl groups. Benzene is most preferred.

Examples of suitable phenolic compounds include diphenols such as catechol, 4-methylcatechol, 5-methylcatechol, resorcinol, 2-methylresorcinol, 5-methylresorcinol, and hydroquinone; biphenyloids such as 4,4'-biphenyldiol and 3,4'-diphenyldiol; and catechol derivatives such as dopa, dopamine, chlorogenic acid, caffeic acid, paracoumaric acid, tyrosine. Catechol, tyrosine and chlorogenic acid are preferred. The amount of phenolic compounds to be added is not particularly limited. However, in general, they are added in a proportion of at least 1 mg, preferably of at least 10 mg, most preferably of 20 to 80 mg, with respect to 100 mg ofESM.

The enzyme component of the ESM includes enzymes capable of oxidizing phenolic compounds, or a medium containing these enzymes. Preferred enzymes include enzymes capable of oxidizing the above mentioned substrates into compounds having a quinone structure; enzymes capable of adding phenolic hydroxyl groups to the substrates and then transforming the resulting product into a compound having a quinone structure; and a medium containing these enzymes.

Examples of such enzymes include polyphenol oxidases, monophenol oxidases, catechol oxidases, peroxidases, oxidases forming hydrogen peroxide and plant substances containing oxidative enzymes.

More specifically, they include a phenolase, an urushiol oxidase, glucose oxidase, laccase, and tyrpsinase.

Amongst them, preferred polyphenol oxidases include laccase, urushiol oxidase and phenolase.

Alternatively, preferred monophenol oxidases include tyrosinase.

Examples of the plants containing oxidative enzymes include at least one plant selected from the group consisting of fruits, vegetables, nuts, herbs, spices teas, marine algae, bacteria and fungi. They also include mushrooms of genera Agaricus and Boletus such as *Agaricus bisporus* and *Boletus pulverulentus.*

More specifically, such plants include apples, bananas, pears, strawberries, persimmons, pineapples, young citrus fruits, grapes, apricots, peaches, plums, papayas, quinces, avocados, mangos, cherries, melons, loquats, figs, prunes, kiwis, blueberries, black berries, raspberries, cranberries, black currants, burdocks, eggplants, tomatoes, mugworts, lotus roots, lettuces, cabbages, beets, hops, parsnips, spinach, radishes, turnips, cauliflower, chicory, onions, celery, carrots, asparagus, green chili, horseradishes, wasabis, ginger, aloes, green peppers, barley, wheat, corns, alfalfa, malts, broad beans, soy beans, adzuki beans, red beans, green beans, sugar beans, French beans, mung beans, potatoes, sweet potatoes, sugar canes, yams, taros, tobaccos, olives, lacquer tree latex and chrysanthemums.

The prepared enzymes may be used alone or as a mixture. They are not limited to commercially available enzymes. They may be prepared according to a known method, such as enzyme-acetone powdering or lyophilization.

The amount of enzymes to be added depends on its enzymatic activity, but should be sufficient to allow the enzyme to oxidize phenolic compounds. For the reaction to be accelerated, the enzyme is preferably added in an amount that provides more than 100 units of enzyme activity per 100 mg ofESM.

The above enzyme activity is defined as one unit, when the absorption value at OD 265 nm increases by 0.001 after substrate L-DOPA is oxidized at 25°C and pH 6.5 for one minute.

The FOF substances added in the deodorant composition are preferably chosen from the group consisting of volatile compounds having a molecular weight lower than 500 Dalton, an essential oil and an extract.

The perfumery substances can be evaluated for their "freshness", "cleanliness" and "pleasant feeling", and respectively rated from zero points to 100 points. The FOF substances of the invention obtain a rating exceeding 50 points for each item according to the above evaluation tests. Preferably, the FOF substances of the invention include no flavor which contains sulfur- or nitrogen-bearing compounds as an essential perfumery component.

Examples of flavors containing sulfur- or nitrogen-bearing compounds are listed in Table 1.

**Table 1**

| Flavors | S or N bearing compounds found in the flavor |
|---|---|
| Spring or Welsh onion flavor | dipropyl disulfide |
| Chiboul flavor | dipropyl disulfide |
| Scallion flavor | dipropyl disulfide; allylmethyl disulfide |
| Leek flavor | dimethyl disulfide; dimethyl trisulfide |
| Garlic flavor | diallyl trisulfide; diallyl disulfide |
| Corn flavor | dimethyl sulfide |
| Onion flavor | dipropyl disulfide; dipropyl trisulfide |
| Sesame flavor | pyrazines |
| Crab flavor | dimethyl sulfide |
| Cuttlefish flavor | pyrazines |
| Shrimp or prawn flavor | amines |
| Coffee flavor | furfuryl mercaptan |
| Mango flavor | dimethyl sulfide |
| Passion fruit flavor | oxathian |
| Cheese flavor | dimethyl sulfide; methional |

The "essential perfumery component" mentioned above is defined as a component which is present in the fragrance, flavor, essential oil or plant extracts, and characterises the quality and features of perfumery.

The FOF substances of the invention are commonly used by combining a plurality of perfume components, essential oil and/or extracts. However, they can also be used as a sole perfume component.

The freshness, cleanliness and pleasant feeling of the fragrance and flavor are evaluated as follows.

The fragrance (or flavor) and ESM were placed in a bottle in a weight proportion of one to one, and mixed therein. A panel of 3 to 5 persons tested the flavor near the neck of the bottle, and rated the mean results from zero to 100 points for three criteria, freshness, cleanliness and pleasant feeling. Rating standards are defined in Table 2 (freshness), Table 3 (cleanliness) and Table 4 (pleasant feeling).

**Table 2**

| Scores | Evaluation |
|---|---|
| 0 | No freshness |
| 25 | Little freshness |
| 50 | Partial freshness |
| 75 | Strong freshness |
| 100 | Very strong freshness |

**Table 3**

| Scores | Evaluation |
|---|---|
| 0 | No cleanliness |
| 25 | Little cleanliness |
| 50 | Partial cleanliness |
| 75 | Strong cleanliness |
| 100 | Very strong cleanliness |

**Table 4**

| Scores | Evaluation |
|---|---|
| 0 | No pleasant feeling |
| 25 | Little pleasant feeling |
| 50 | Partial pleasant feeling |
| 75 | Strong pleasant feeling |
| 100 | Very strong pleasant feeling |

An appropriate fragrance according to the invention is prepared from at least one fragrance chosen from the group consisting of citrus-type fragrance, fruit-type fragrance, flower-type (or floral-type) fragrance, musk-type fragrance, wood-type (or "woody"-type) fragrance, greenery-type (or green-type) fragrance, spice-type fragrance, herb-type fragrance and mint-type fragrance.

An appropriate flavor according to the invention is prepared from at least one fragrance chosen from the group consisting of citrus-type flavor, mint-type flavor, fruit-type flavor, vanilla-type flavor, spice-type flavor, flower-type flavor and, Perilla-type flavor.

In the above expression, the term "citrus-type fragrance (or flavor)", for example, defines "citrus fragrance (or flavor)" and "citrus-like fragrance (or flavor)".

The amount of FOF to be added to the deodorant composition depends on the use purpose. Usually, its amount must be sufficient to remove the last remaining trace of odors which could not be inactivated by the ESM. Its amount is preferably at least about 0.1% by weight, more preferably at least about 1% by weight, with respect to the amount of deodorant composition.

Examples of the volatile compounds having a molecular weight below 500 Dalton, which constitute the fragrance of the invention, include: Amyl salicylate, Benzyl acetate, Benzyl salicylate, 1,1,2,3,3-pentamethyl-2,3,5,6,7-pentahydroinden-4-one, 2,6,6,8-tetramethyltricyclo[5.3.1.0<1,5>]undecan-8-ol, Citronellol, tricyclo[5.2.1.0<2,6>]dec-4-en-8-yl acetate, tricyclo[5.2.1.0<2,6>]dec-4-en-8-yl propanoate, 2,6-dimethyloct-7-en-2-ol, 2,6-dimethylheptan-1-ol, Phenoxybenzene, 4,6,6,7,8,8-hexamethyl-6,7,8-trihydrocyclopenta[1,2-g]isochromane, Galbanum oil, Geranyl acetate, Geranyl nitrile, 3-ethoxy-1,1,5-trimethylcyclohexane, Hexyl cinnamic aldehyde, Hexyl salicylate, Iso-Bornyl acetate, 1-(3,4,10,10-tetramethylbicyclo[4.4.0]dec-5-en-3-yl)ethan-1-one, 2-((2Z)pent-2-enyl)-3-methylcyclopent-2-en-1-one, 3-[4-(tert-butyl)phenyl]-2-methylpropanal, Linalool, Methyl 2-aminobenzoate, (1E)-1-(6,6-dimethyl-2-methylenecyclohexyl)pent-1-en-3-one methyl 2-((1E)-1-aza-8-hydroxy-4,8-dimethylnon-1-enyl)benzoate, 2-nonynal dimethyl acetal, 2-phenylethan-1-ol, α-Terpineol, 1-((6S,1R)-2,2,6-trimethylcyclohexyl)hexan-3-ol, 2-(tert-butyl)cyclohexyl acetate, 4-(tert-butyl)cyclohexyl acetate, 2-methoxynaphthalene, 1-(2,6,6,8-tetramethyltricyclo[5.3.1.0<1,5>]undec-8-en-9-yl)ethan-1-one, Acetyl iso-eugenol, Allyl amyl glycolate, 1,6,10,10-tetramethyl-5-oxatricyclo[7.4.0.0<2,6>]tridecane, (1S,2R,6R)-1,6,10,10-tetramethyl-5-oxatricyclo[7.4.0.0<2,6>]tridecane, Amyl α-cinnamic aldehdye, Anisic aldehyde, Benzyl acetate, Bergamot oil, 7-methyl-2H,4H-benzo[b]1,4-dioxepin-3-one, Cinnamic alcohol, Citronellol, 2-methyl-3-[4-(methylethyl)phenyl]propanal, 2-oxabicyclo[4.4.0]decan-3-one, (2E)-1-(2,6,6-trimethylcyclohex-3-enyl)but-2-en-1-one, 2,6-dimethyloct-7-en-2-ol, 1,1-dimethyl-2-phenylethyl acetate, 2,6-dimethylheptan-1-ol, Eugenol, 2-oxacyclohexadecan-1-one, 3-(4-ethylphenyl)-2,2-dimethylpropanal, Geraniol, methyl 2-(3-oxo-2-pentylcyclopentyl)acetate, Hexyl acetate, Hexyl salicylate, 2H,4H,4aH,9aH-indano[2,1-d]1,3-dioxane, (3E)-4-(2,6,6-trimethylcyclohex-2-enyl)but-3-en-2-one, (3E)-4-(6,6-dimethyl-2-methylenecyclohexyl)but-3-en-2-one, 1-(3,4,10,10-tetramethylbicyclo[4.4.0]dec-5-en-3-yl)ethan-1-one, 2-((2Z)pent-2-enyl)-3-methylcyclopent-2-en-1-one, 4-(4-hydroxy-4-methylpentyl)cyclohex-3-enecarbaldehyde, 3-[4-(tert-butyl)phenyl]-2-methylpropanal, (5E)-2,6-dimethylhept-5-enal, Methyl chavicol, (1E)-1-(6,6-dimethyl-2-methylenecyclohexyl)pent-1-en-3-one, 2,5-dioxacycloheptadecane-1,6-dione, Trans-2-Tridecenal, Phenyl ethyl acetate, Phenyl ethyl alcohol, Styrallyl acetate, Dimethyl cyclohexenal, 5-heptyl-3,4,5-trihydrofuran-2-one, 2-(tert-butyl)cyclohexyl acetate, α-Fenchyl alcohol, 1-Decanal, 2,6-dimethyloct-7-en-2-ol, 4,6,6,7,8,8-Hexamethyl-G,7,8-trihydrocyclopenta[1,2-g]isochromane, Benzyl benzoate, Methyl 2-(3-oxo-2-pentylcyclopentyl)acetate, 3,7-Dimethyl-2,6-octadien-1-al, Linalyl acetate, Petigrain oil, Lemon oil, Lime oil, Geranyl nitrile, Tetrahydrolinalool, vanillin, caryophyllene. Lemon oil, Orange oil, citrus oil, cedarwood oil, Bois de rose oil, citronella oil, patchouli oil, eucalyptus oil, bay oil, grapefruit oil, mandarin oil, sandalwood oil, juniper berry oil, rose oil, ylang oil, tangerine oil, geranium oil and Limonene.

Examples of the volatile compounds having a molecular weight below 500 Dalton, which constitute the flavor of the invention, include: menthol, iso-pulegole, eucaryptol, p-mentha-3,8-diol, vanilyl butyl ether, apple oil, apricot oil, cassia oil, cinnamic aldehyde, allyl hexanoate, isoamyl acetate, amyl alcohol, anethole, benzaldehyde, benzyl acetate, isobutyl acetate, butyl butyrate, isobutyl butyrate, camphor, carvone, β-caryophyllene, cinnamaldehyde, cinnamyl alcohol, citral, citronellyl actate, cumin aldehyde, cymene, decalactone, decanal, diacetyl, ethyl acetoacetate, ethyl anthranilate, ethyl butylate, ethyl hexanoate, ethyl lactate, ethyl 2-methylbutylate, ethyl salicylate, ethyl vanillin, ethyl menthol, eugenol, iso-eugenol, furfural, furfuryl alcohol, geraniol, geranyl acetate, hexanal, hexenal, hexynyl acetate, hexyl alcohol, ionone, irone, limonene, linalool, linalyl acetate, maltol, menthol, menthone, methyl acetate, methyl anthranilate, methyl cinnmate, methyl salicylate, nerol, nerolidol, nonalactone, nonanal, octalactone, octanal, octanol, octenol , octyl acetate, phenyl acetate, phenetyl alcohol, pinenes, piperanal, propyl acetate, thymol, undecalactone,

Examples of essential oil constituting the flavor of the invention include: lemon oil, orange oil, grape fruit oil, mint oil, peppermint oil, apple oil, apricot oil, cassia oil, camphor, anise oil, anise star oil, basil oil, bay leaves west Indian oil, bois de rose oil, buchu leaves oil, cardamon seed oil, cassia bark oil, chmomile flower Roman oil, cinnamon bark oil, cinnamon leaf oil, citronella oil, clove bud oil, cognac green oil, coriander oil, cubebs oil, cumin oil, eucalyptus oil, fennel sweet oil, garlic oil, ginger oil, grapefruit oil.

The composition of an example of citrus-type fragrance is shown in Table 5.

**Table 5**

| Citrus-type fragrance materials | Ratio (%) |
|---|---|
| α-Fenchyl alcohol | 0.2 |
| 1-Decanal | 1.0 |
| Citronellol | 3.8 |
| 2,6-dimethyloct-7-en-2-ol | 3.4 |
| 4,6,6,7,8,8-Hexamethyl-6,7,8-trihydrocyclopenta[1,2-g]isochromane | 2.5 |
| Benzyl benzoate | 2.5 |
| Methyl 2-(3-oxo-2-pentylcyclopentyl)acetate | 1.0 |
| 3,7-Dimethyl-2,6-octadien-1-al | 32.0 |
| Linalyl acetate | 0.6 |
| Linalool | 2.0 |
| Styrallyl acetate | 2.0 |
| Petigrain oil | 0.2 |
| Geraniol | 10.0 |
| Lemon oil | 7.0 |
| Lime oil | 27.0 |
| Butylhydroxytoluene | 2.4 |
| Geranyl nitrile | 2.4 |

The composition of an example of fruit- and flower-type fragrance is shown in Table 6.

**Table 6**

| Fruit- and flower-type fragrance materials | Ratio (%) |
|---|---|
| 1-(2,6,6,8-tetramethyltricyclo[5.3.1.0<1,5>]undec-8-en-9-yl)ethan-1-one | 2.0 |
| Acetyl iso-eugenol | 1.0 |
| Allyl amyl glycolate | 1.0 |
| 1,6,10,10-tetramethyl-5-oxatricyclo[7.4.0.0<2,6>]tridecane | 1.0 |
| (1S,2R,6R)-1,6,10,10-tetramethyl-5-oxatricyclo[7.4.0.0<2,6>]tridecane | 0.5 |
| Amyl-α-cinnamic aldehdye | 1.0 |
| Anisic aldehyde | 1.0 |
| Benzyl acetate | 4.5 |
| Benzyl salicylate | 4.5 |
| Bergamot oil | 0.5 |
| 7-methyl-2H,4H-benzo[b]1,4-dioxepin-3-one | 0.5 |
| Cinnamic alcohol | 0.5 |
| Citronellol | 7.0 |
| 2-methyl-3-[4-(methylethyl)phenyl]propanal | 0.5 |
| 2-oxabicyclo[4.4.0]decan-3-one | 1.5 |
| (2E)-1-(2,6,6-trimethylcyclohex-3-enyl)but-2-en-1-one | 0.5 |
| 2,6-dimethyloct-7-en-2-ol | 1.0 |
| 1,1-dimethyl-2-phenylethyl acetate | 4.5 |
| 2,6-dimethylheptan-1-ol | 2.5 |
| Eugenol | 1.0 |
| 2-oxacyclohexadecan-1-one | 3.5 |
| 3-(4-ethylphenyl)-2,2-dimethylpropanal | 1.0 |
| Geraniol | 1.5 |
| methyl 2-(3-oxo-2-pentylcyclopentyl)acetate | 15.5 |
| Hexyl acetate | 0.5 |
| Hexyl salicylate | 5.0 |
| 2H,4H,4aH,9aH-indano[2,1-d] 1,3 -dioxane | 0.5 |
| (3E)-4-(2,6,6-trimethylcyclohex-2-enyl)but-3-en-2-one | 1.0 |
| (3E)-4-(6,6-dimethyl-2-methylenecyclohexyl)but-3-en-2-one | 1.5 |
| 1-(3,4,10,10-tetramethylbicyclo[4.4.0]dec-5-en-3-yl)ethan-1-one | 1.0 |
| 2-((2Z)pent-2-enyl)-3-methylcyclopent-2-en-1-one | 0.1 |
| 4-(4-hydroxy-4-methylpentyl)cyclohex-3-enecarbaldehyde | 3.0 |
| 3-[4-(tert-butyl)phenyl]-2-methylpropanal | 9.0 |
| Linalool | 2.3 |
| (5E)-2,6-dimethylhept-5-enal | 0.3 |
| Methyl chavicol | 0.5 |
| (1E)-1-(6,6-dimethyl-2-methylenecyclohexyl)pent-1-en-3-one | 4.0 |
| 2,5-dioxacycloheptadecane-1,6-dione | 3.5 |
| Trans-2-Tridecenal | 1.0 |
| Phenyl ethyl acetate | 0.5 |
| Phenyl ethyl alcohol | 5.0 |
| Styrallyl acetate | 0.5 |
| Dimethyl cyclohexenal | 0.3 |
| 5-heptyl-3,4,5-trihydrofuran-2-one | 1.0 |
| 2-(tert-butyl)cyclohexyl acetate | 1.5 |
| Total | 100.0 |

The composition of an example of musk-and wood-type fragrance is given in Table 7.

**Table 7**

| Musk- and wood-type fragrance materials | Ratio (%) |
|---|---|
| Amyl salicylate | 3.0 |
| Benzyl acetate | 1.5 |
| Benzyl salicylate | 8.0 |
| 1,1,2,3,3-pentamethyl-2,3,5,6,7-pentahydroinden-4-one | 0.5 |
| 2,6,6,8-tetramethyltricyclo[5.3.1.0<1,5>]undecan-8-ol | 1.5 |
| Citronellol | 3.0 |
| tricyclo[5.2.1.0<2,6>]dec-4-en-8-yl acetate | 0.5 |
| tricyclo[5.2.1.0<2,6>]dec-4-en-8-yl propanoate | 1.5 |
| 2,6-dimethyloct-7-en-2-ol | 3.0 |
| 2,6-dimethylheptan-1-ol | 0.5 |
| Phenoxybenzene | 0.5 |
| Dipropylene glycol | 11.0 |
| 4,6,6,7,8,8-hexamethyl-6,7,8-trihydrocyclopenta[1,2-g]isochromane | 2.5 |
| Galbanum oil | 0.5 |
| Geranyl acetate | 1.0 |
| Geranyl nitrile | 0.5 |
| 3-ethoxy-1,1,5-trimethylcyclohexane | 0.5 |
| Hexyl cinnamic aldehyde | 17.0 |
| Hexyl salicylate | 12.0 |
| Iso-Bornyl acetate | 0.5 |
| 1-(3,4,10,10-tetramethylbicyclo[4.4.0]dec-5-en-3-yl)ethan-1-one | 3.0 |
| 2-((2Z)pent-2-enyl)-3-methylcyclopent-2-en-1-one | 0.5 |
| 3-[4-(tert-butyl)phenyl]-2-methylpropanal | 3.5 |
| Linalool | 3.5 |
| methyl 2-aminobenzoate | 0.5 |
| (1E)-1-(6,6-dimethyl-2-methylenecyclohexyl)pent-1-en-3-one | 4.0 |
| methyl N-3,7-dimethyl-7-hydroxyoctylidene-anthranilate | 0.5 |
| 2-nonen-1-al dimethyl acetal | 0.5 |
| 2-phenylethan-1-ol | 3.0 |
| α-terpineol | 3.5 |
| 1-((6S,1R)-2,2,6-trimethylcyclohexyl)hexan-3-ol | 0.5 |
| 2-(tert-butyl)cyclohexyl acetate | 3.5 |
| 4-(tert-butyl)cyclohexyl acetate | 4.0 |
| 2-methoxynaphthalene | 0.5 |

The composition of an example of greenery- and flower-type fragrance is given in Table 8.

**Table 8**

| Green- and flower-type fragrance materials | Ratio (%) |
|---|---|
| 1-Hydroxy citronellol | 2.0 |
| Undecylic aldehyde | 0.1 |
| Octyl aldehyde | 0.1 |
| Dodecyl aldehyde | 0.1 |
| γ-Undeca lactone | 0.5 |
| Anethole | 0.2 |
| (2E)-4-((1R)-2,2,3-trimethylcyclopent-3-enyl)-2-ethylbut-2-en-1-ol | 0.2 |
| 1-Citronellol | 1.0 |
| p-Cresyl phenyl acetate | 0.3 |
| Tricyclodecenyl propionate | 4.0 |
| Dimethylbenzyl carbinyl acetate | 3.0 |
| Dimethylbenzyl carbinyl alcohol | 2.0 |
| 2,6-Dimethylheptan-2-ol | 3.0 |
| Eugenol | 1.0 |
| (3E)-4-(6,6-dimethyl-2-methylenecyclohexyl)but-3-en-2-one | 4.0 |
| 4,6,6,7,8,8-hexamethyl-6,7,8-trihydrocyclopenta[1,2-g]isochromane and its isomers in benzyl benzoate | 10.0 |
| Geraniol | 2.0 |
| Hexylcinnamic aldehyde | 5.0 |
| Hexyl salicylate | 8.0 |
| 1-(3,4,10,10-tetramethylbicyclo[4.4.0]dec-5-en-3-yl)ethan-1-one and its isomers | 4.5 |
| Lime oil | 1.0 |
| Teterahydro linalool | 1.0 |
| Tetrahydro mugol | 1.0 |
| Phenylethyl alcohol | 12.0 |
| 4-methyl-2-(2-methylprop-1-enyl)-2H-3,4,5,6-tetrahydropyran | 0.1 |
| Dipropylene glycol | 33.9 |
| Total | 100.0 |

The composition of an example of mint-type fragrance is listed in Table 9.

**Table 9**

| Fragrance Materials | Ratio (%) |
|---|---|
| Mentha arvensis oil | 32.0 |
| 1-Menthol | 16.0 |
| Peppermint oil | 20.0 |
| α-Damascone | 0.2 |
| Isoamyl acetate | 1.0 |
| Lemon oil | 6.0 |
| Dipropylene glycol | 24.8 |
| Total | 100.0 |

Now, the composition of an example of peppermint-type flavor is given in Table

**Table 10**

| Peppermint-type flavor materials | Weight % |
|---|---|
| Ethylsalicylate | 10.0 |
| Dried bonito oleoresin | 1.0 |
| 1-Menthol | 30.0 |
| Peppermint oil | 54.0 |
| Mentha arvensis oil | 5.0 |
| Total | 100.0 |

The composition of an example of mint-type flavor is given in Table 11.

**Table 11**

| Mint-type flavor materials | Weight % |
|---|---|
| Peppermint oil | 20.0 |
| 1-Menthol | 40.0 |
| Anethole | 6.0 |
| Lemon oil | 1.0 |
| Spearmint oil | 10.0 |
| 1-Carvone | 23.0 |
| Total | 100.0 |

The composition of an example of lemon-type flavor is given in Table 12.

**Table 12**

| Materials | Weight % |
|---|---|
| Anethole | 2.0 |
| Eucalyptus oil | 10.0 |
| Lemon oil | 1.0 |
| 1-Carvone | 25.0 |
| Ethanol | 27.0 |
| 1-Menthol | 25.0 |
| Spearmint oil | 10.0 |
| Total | 100.0 |

The composition of an example of mint powder-type flavor is given in Table 13.

**Table 13**

| Mint powder-type flavor materials | Weight % |
|---|---|
| Peppermint oil | 10.0 |
| Arabic gum | 30.0 |
| Dextrin | 60.0 |
| Total | 100.0 |

The composition of an example of perilla-type flavor is given in Table 14.

**Table 14**

| Perilla-type flavor materials | Weight % |
|---|---|
| Perilla leaves oil | 10.0 |
| Fatty acid esters having a medium chain length | 90.0 |
| Total | 100.0 |

As mentioned supra, the deodorant composition of the invention is applied to malodorous objects or articles, as well as to mammals or birds.

Examples of the mammals include human, pets e.g. dogs, cats, birds, and domestic animals e.g. cows, pigs and horses.

The malodorous objects may be malodorous mouths, sweat skin, a fridge odor, industrial wastes, kitchen refuse, garbage, or feces or animal droppings. They further include the foods generating special odors such as meats, fish and natto (fermented soy beans).

The applying methods are not limited to any specific means. Typically, the deodorant composition is formulated into a ready-to-spray preparation, and sprayed onto the malodorous objects by any spraying means.

The deodorant composition of the invention may be used directly onto the malodorous objects. However, it can also be mixed beforehand with foods or detergents, so that malodors can be prevented from leaking out.

Examples of the preferred products or articles, with which the inventive deodorant composition are used, include: a food, a candy, a cookie, a lozenge, a chewing gum, a mouthwash, a toothpaste, an oral care product, a household cleaning product, a toilet cleaner, a wet towel, a bath room cleaner, a kitchen cleaner, a shoe box spray, a hair care product, a shampoo, a conditioner, a hair spray, a skin care product, a cosmetic, a toiletry, a personal care product, a professional strength sanitizing product, an air-freshener, a medicine, a pet food, a garbage spray, an agricultural product, a pet food, a litter care product, and a pet product.

The deodorant composition may also be supplemented with additives. Examples of the preferred additives include: a solvent, a glycol, a glyceride, a grain powder, a lipid, a polymer, a protein, a starch, a polysaccharide, a surfactant, a gum, a silica gel, a powder, and a capsule.

The deodorizing composition is preferably used with known antimicrobial agents such as benzoic acid, sodium benzoate, chlorhexidine, triclosan, quarterly ammonium salts, thymol, and carvacrol, and/or anti-oxidant agent such as BHT, BHA, vitamin E and vitamin C. In this manner, the deodorizing composition and its activity are prevent from deterioration. Moreover, these additives obviate malodors generated by microbial putrefaction. Further, the deodorizing composition is preferably formulated into a capsule by any known encapsulation technology, so that its activity can be delivered on demand. It is also possible to add thereto other commonly used additives

Five types of fragrance and five types of flavors were evaluated by a group of 3 to 5 panelists using a rating scale ranging from 0 point to 100 points.

FOF were evaluated for three criteria, namely freshness, cleanliness and pleasant feeling. Ratings between 50 points and 100 points are considered as satisfactory.

The results of the evaluation for the fragrance and for the flavor are respectively given in Table 15 and Table 16.

**Table 15**

| Fragrance | Freshness index | Cleanliness index | Pleasant feeling index |
|---|---|---|---|
| Citrus-type | 85 | 82 | 82 |
| Fruit- and floral-type | 67 | 79 | 81 |
| Musk- and wood-type | 71 | 75 | 75 |
| Greenery- and floral-type | 68 | 76 | 81 |
| Mint-type | 91 | 85 | 79 |

**Table 16**

| Flavor | Freshness Index | Cleanliness Index | Pleasant feeling Index |
|---|---|---|---|
| Peppermint-type | 78 | 78 | 76 |
| Mint-type | 80 | 88 | 79 |
| Lemon-type | 93 | 80 | 95 |
| Mint powder-type | 80 | 88 | 74 |
| Perilla-type | 85 | 85 | 85 |

The deodorizing effect of the deodorant composition was evaluated by a group of five panelists. The results of the evaluation are rated into 5 levels of score.

**Table 17**

| Scores | Foul-smelling rate |
|---|---|
| 0 | No malodor smelling |
| 1 | Hardly smelling |
| 2 | Weak but identifiable smelling |
| 3 | Easily identifiable smelling |
| 4 | Strong smelling |
| 5 | Very strong smelling |

### Embodiment 1 (E1)

In the present test, the capacity of the deodorant composition to eliminate urine odor was evaluated. A deodorant composition sample was prepared as follows. Substrate phenols were obtained from raw coffee beans as follows. The raw beans were ground by a grinder and extracted with hot water at 85 to 95°C for 2 hours. The extract was filtered, and the filtrate was condensed and dried, to give a phenol substrate. The phenol substrate contained approximately 30% by weight of polyphenolic compounds.

Enzymes were obtained from the burdock. The burdock was freeze-dried and ground to give an enzyme powder. Likewise, an active acetone-powder enzyme was obtained by grinding the burdock in ice-frozen acetone, filtering off the acetone phase, and drying up the enzyme-containing residue. The specific enzyme activity was approximately 70units/mg.

The substrate and the enzymes were mixed in a proportion of 1 : 1 (w/w), so as to obtain a sample ESM-1.

10 ml of human urine, 15 mg of ESM-1 (0.15%) and 10µlof citrus-type fragrance (0.1%) were mixed in a vial bottle, and incubated under shaking at 25°C, for 15 min or 1 week.

### Comparative Example 1 (CE1)

10µl of water and 10 ml of human urine were mixed in a bottle, and the whole solution was reacted as described for E1.

### Comparative Example 2 (CE2)

10 ml of human urine and 15 mg of ESM-1 (0.15%) were mixed in a bottle, and the whole solution was reacted as described for E1.

### Comparative Example 3 (CE3)

10 ml of human urine and 10µl of citrus-type fragrance (0.1%) were mixed in a bottle, and the whole solution was reacted as described for E1.

### Comparative Example 4 (CE4)

10ml human urine and 50µl of citrus-type fragrance (0.5%) were mixed in a bottle, and the whole solution was reacted as described for E1.

The deodorizing effect of the deodorant composition of E1, CE1, CE2, CE3 and CE4 were evaluated organoleptically by a group of 5 panelists.

The results obtained after 15 minutes of incubation are given in Table 18.

**Table 18**

| Samples | Freshness index | Cleanliness index | Pleasant feeling index | Malodor (Urine) smelling index | Evaluation |
|---|---|---|---|---|---|
| CE1 | OOE | OOE | OOE | 5.0 | Very strong urine odor |
| CE2 | OOE | OOE | OOE | 0.6 | Only a weak urine odor; strong urine odor disappears, but foul-smelling still lingers |
| CE3 | 25 | 16 | 16 | 3.8 | Initially fragrance odor, then strong urine odor. No meaningful deodorizing effect perceived. |
| CE4 | 25 | 16 | 16 | 3.2 | Initially fragrance odor, then strong urine odor. No meaningful deodorizing effect perceived. |
| E1 | 85 | 82 | 82 | 0 | No urine-smelling; initial fragrance retained. |

In the above and following tables, "00E" indicates that a meaningful evaluation could not be carried out.

The results obtained after one week of incubation are given in Table 19.

**Table 19**

| Samples | Freshness index | Cleanliness index | Pleasant feeling index | Malodor (Urine) smelling index | Evaluation |
|---|---|---|---|---|---|
| CE1 | OOE | OOE | OOE | 5.0 | Very strong urine odor |
| CE2 | OOE | OOE | OOE | 0.6 | Only a weak urine odor; strong urine odor disappears, but foul-smelling still lingers. |
| CE3 | 8 | 0 | 8 | 4.4 | Initially fragrance odor, then strong urine odor. No meaningful deodorizing effect perceived. |
| CE4 | 16 | 16 | 16 | 3.4 | Initially fragrance odor, then strong urine odor. No meaningful deodorizing effect perceived. |
| E1 | 85 | 82 | 82 | 0 | No urine smelling; initial fragrance retained. |

The above results indicate that, in the deodorant composition of the invention, the citrus-type fragrance and ESM react synergistically to remove urine odor. Even when the deodorizing composition merely containing polyphenolic compounds and enzymes is used, urine-smelling is effectively reduced. However, bad smelling still lingers. When the fragrance material alone is used, no meaningful deodorizing effect is observed, and initial fragrance partially disappears.

### Embodiment 2 (E2)

In the present test, the capacity of the deodorant composition to eliminate urine odor from a cat litter was evaluated. A deodorant composition sample was prepared as follows. A phenol substrate was obtained from commercially available rosemary extracts (manufactured by TOKYO TANABE SEIYAKU KABUSHIKI KAISHA). The proportion of polyphenol compounds was about 6% by weight. Enzyme powder was obtained by freeze-drying bananas and grinding the dried bananas. The specific enzyme activity was 60 unit/mg.

The substrate and the enzymes were mixed in a proportion of 1 : 1 (w/w), so as to obtain a composition sample (ESM-2).

10 ml of water, 10 µl of mint-type fragrance (0.1%) and 15 mg of ESM-2 (0.15%) were mixed with a cat litter including cat urine, and the whole mixture was incubated at 34°C for one hour.

### Comparative Example 5 (CE5)

As a control, 10 ml of water and a cat litter including cat urine were mixed, and the whole mixture was incubated under the same conditions as above.

### Comparative Example 6 (CE6)

10ml of water, 15mg of ESM-2 (0.15%) and a cat litter including cat urine were mixed, and the whole mixture was incubated under the above conditions.

### Comparative Example 7 (CE7)

10ml of water, 10µl of mint-type fragrance (0.1%) and a cat litter including cat urine were mixed, and the whole mixture was incubated under the same conditions.

The deodorizing effect of E2, CE5, CE6 and CE7 was evaluated by a group of 5 panelists. The results are given in Table 20.

**Table 20**

| Samples | Freshness index | Cleanliness index | Pleasant feeling index | Malodor (Urine) smelling index | Evaluation |
|---|---|---|---|---|---|
| CE5 | OOE | OOE | OOE | 5.0 | Very strong urine odor |
| CE6 CE7 | OOE | OOE | OOE | 0.7 | Only a weak urine odor; strong urine odor disappears, but foul-smelling still lingers. |
| | 40 | 28 | 35 | 4.0 | fragrance odor at first, then strong urine odor. No meaningful urine-deodorizing or -masking effect perceived. |
| E2 | 91 | 85 | 79 | 0 | No urine odor smelled. Initial mint fragrance retained. |

The results show that the deodorizing composition of the present invention effectively eliminates the urine odor in the cat litter. When an ESM is used alone (without using the FOF substances), it can reduce urine odor, but not a lingering foul-smelling. Use of a fragrance alone cannot remove urine odor effectively, and initial fragrance partially disappears.

### Embodiment 3 (E3)

In the present test, the capacity of the deodorant composition to eliminate a bleach odor from a laundry was evaluated. 10cm x 10cm of clothes were soaked in hypochlorite bleach for 2 minutes, and rinsed once with water. The clothes were washed with the a powder detergent composition, and then rinsed with water.

The phenol substrate used was commercially available sun flower seeds extracts (manufactured by DAINIPPON INK KAGAKU KOGYO KABUSHIKI KAISHA). The proportion of polyphenolic compounds in the phenol substrate was about 6% by weight.

Enzymes were obtained as follows. Prematurely harvested apples were ground in ice-frozen acetone. The obtained suspension was filtered off, and the residue was washed with another ice-frozen acetone. The residue was then dried in vacuo to obtain acetone-powder enzymes. Specific enzyme activity was approximately 70 units/mg.

The substrate and the enzymes were mixed in a proportion of 1 : 1 (w/w) to prepare an sample ESM-3.

The powder detergent product was prepared by adding 1% by weight of musk- and wood-type fragrance and 1% by weight of ESM-3 to the detergent product.

The composition of an example of powder detergent product is shown in Table 21.

**Table 21**

| Materials | Weight % |
|---|---|
| Sodium C-12 - C-18 pareih sulfate | 15.0 |
| Sodium carbonate | 15.0 |
| Sodium meta-silicate | 13.0 |
| Sodium citrate | 15.0 |
| Carboxy methyl cellulose | 2.0 |
| Sodium sulfate | 38.0 |
| Musk- and wood-type fragrance | 1.0 |
| ESM-3 | 1.0 |
| Total | 100.0 |

The composition of the bleach used is given in Table 22.

**Table 22**

| Materials | Weight % |
|---|---|
| Sodium hypochlorite | 6.0 |
| Cocoyl dimethyl amine oxide | 5.0 |
| Potassium pyrophosphate | 3.0 |
| Water | 86.0 |
| Total | 100.0 |

### Comparative Example 8 (CE8)

A powder detergent product was prepared without ESM-3 and fragrances, and the prepared product was applied to cloth washing as described for E3..

### Comparative Example 9 (CE9)

A powder detergent product was prepared with 1% of ESM-3, and the prepared product was applied to cloth washing as described for E3.

### Comparative Example 10 (CE10)

A powder detergent product was prepared with 1% of musk- and wood-type fragrance, and the prepared product was applied to cloth washing as described for E3.

Evaluation tests were carried out by a group of 5 panelists. The results of the evaluation tests are given in Table 23.

**Table 23**

| Samples | Freshness index | Cleanliness index | Pleasant feeling index | Bleach malodor index | Evaluation |
|---|---|---|---|---|---|
| CE-8 | OOE | OOE | OOE | 4.7 | Very strong bleach odor |
| CE-9 | 28 | 25 | 29 | 0.3 | Only a weak bleach odor. Strong deodorizing effect. |
| CE-10 | 28 | 25 | 29 | 3.3 | Initially fragrance odor, then bleach odor; no meaningful deodorizing effect perceived. |
| E3 | 71 | 75 | 75 | 0 | No bleach odor. Initial fragrance remains. |

The results show that the bleach odor of clothes is eliminated when washed with a detergent admixed with the deodorizing composition of the present invention. The use of ESM-3 (containing polyphenolic compounds and enzymes) alone reduces the bleach odor. However, traces of odor still remain. The musk- and wood-type fragrance alone exhibits no meaningful deodorizing effect of the bleach. Moreover, the initial musk- and wood-type fragrance partly disappears.

### Embodiment 4 (E4)

In the present test, the capacity of the deodorant composition to neutralize a bodily odor was evaluated. An aqueous solution of 0.25% butyric acid was mixed with scraps from an antiperspirant stick. Butyric acid has a typical cheese- and sweat-type odor.

The composition of an example of antiperspirant product is given in Table 24.

**Table 24**

| Materials | Weight % |
|---|---|
| PEG-7 glyceryl cocoate | 2.0 |
| Hydrogenated oil | 5.0 |
| Myristyl myristate | 15.0 |
| Cyclomethicone | 33.5 |
| Stearyl alcohol | 20.0 |
| Stearyl isononenoate | 3.0 |
| Aluminum chlorohydrate | 20.0 |
| Fruit-and floral-type fragrance | 0.5 |
| ESM-4 | 1.0 |
| Total | 100 |

A phenol substrate was obtained from grape pericarps as follows. The grape pericarps (strain Campbell) were added into ethanol, and stirred for 2 hours at 70°C. The extracts obtained were condensed to dryness, and solved in water. The water solution was filtered, and the filtrate was adsorbed on XAD-2 (manufactured by ORGANO KABUSHIKI KAISHA) filled in a column. The column was washed with water, and eluted with methanol. The methanol eluate was then condensed to dryness, to yield grape pericarp extracts. The latter contained about 50% by weight of polyphenolic compounds.

The enzymes used were commercially available tyrosinase obtained from mushrooms (product of SIGMA CHEMICAL CO.).

The substrate and the enzymes were mixed in a proportion of 10 : 1 (w/w) to obtain a sample ESM-4. The antiperspirant product contained 1% by weight of ESM-4 and 0.5% by weight of fruit- and floral-type fragrance.

### Comparative Example 11 (CE11)

A antiperspirant product was prepared without ESM-4 and fragrances, and the prepared product was treated as described for E4.

### Comparative Example 12 (CE12)

A antiperspirant product was prepared with 1% of ESM-4 and fragrances, and the prepared product was treated as described for E4.

### Comparative Example 13 (CE13)

A antiperspirant product was prepared with 0.5% of fruit- and floral-type fragrance, and the prepared product was treated as described for E4..

Evaluation tests were performed by a group of 3 panelists. The results of these tests are given in Table 25.

**Table 25**

| Samples | Freshness index | Cleanliness index | Pleasant feeling index | Sweat malodor | Evaluation |
|---|---|---|---|---|---|
| CE11 | OOE | OOE | OOE | 5.0 | Very strong butyric acid odor |
| CE12 | OOE | OOE | OOE | 1.7 | Only a weak butyric acid odor. Strong deodorizing effect. |
| CE13 | 13 | 18 | 21 | 3.3 | Initially fragrance, then butyric acid odor felt. No significant deodorizing effect. |
| E4 | 67 | 79 | 81 | 0 | No butyric acid odor. Initial fragrance remained. |

According to the results, butyric acid odor, which remains after the antiperspirant product was used, is effectively removed by the deodorizing composition of the invention. Use of sample ESM-4 (comprising the polyphenolic compounds and the enzymes) alone reduces sweat odor. However, some foul smelling still remains. Conversely, use of fruit- and floral-type fragrance alone shows no meaningful deodorizing effect. Moreover, initial fruit- and floral-type fragrance partially disappears.

### Embodiment 5 (E5)

In the present embodiment, a cold permanent wave product was prepared, and tested for its capacity of deodorizing a remaining permanent odor.

1.8 g of tress of false hair were dipped or soaked in 50 ml of first solution for 30 minutes. The first solution was prepared by adjusting a 6 wt % thioglycolic acid water solution to pH 9.3 with ammonia water.

The tress of false hair was wiped with papers, and washed once with 100 ml of water.

The hair was dipped in 50 ml of second solution (5% potassium bromate water solution) for 20 minutes.

The hair was wiped with papers, and dipped in 100 ml of ESM-5 containing shampoo solution for 5 minutes.

The hair was wiped with papers, and washed once with 100 ml of water.

The hair was wiped with papers, and subjected to evaluation tests.

A phenol substrate was obtained by grinding burdocks. The ground burdocks were extracted with ethanol under reflux for 2 hours. The extracts were filtered, and the filtrate was condensed to dryness to obtain a burdock extract. The latter contained about 1.5 % by weight of polyphenolic compounds.

The enzymes used were laccases obtained from urushi (products of SIGMA CHEMICAL CO.).

The substrate and the enzymes were mixed in a proportion of 1: 1 (w/w), to obtain a sample ESM-5.

A prepared cold permanent wave product contained 0.5 % by weight of greenery- and floral-type fragrance and 2.0 % by weight of ESM-5.

The composition of an example of cold permanent wave product is given in Table 26.

**Table 26**

| Materials | Weight % |
|---|---|
| Sodium lauryl sulfate | 40.0 |
| Sodium cocoamphoacetate | 10.0 |
| Cocamide DEA | 2.0 |
| Butylene glycol | 2.0 |
| Citric acid | 0.35 |
| Sodium chloride | 0.1 |
| Methylparaben | 0.2 |
| Propylparaben | 0.1 |
| Tetrasodium EDTA | 0.1 |
| Greenery- and floral-type fragrance | 0.5 |
| ESM-5 | 2.0 |
| Purified water | Balance |
| Total | 100.0 |

### Comparative Example 14 (CE14)

A cold perm wave product was prepared without ESM-5 and fragrance, and the prepared product was treated as described for E5.

### Comparative Example 15 (CE15)

A cold perm wave product was prepared with 2% of ESM-5, and the prepared product was treated as described for E5.

### Comparative Example 16 (CE16)

A cold perm wave product was prepared with 0.5% of greenery- and floral-type fragrance, and the prepared product was treated as described for E5.

The samples E5, CE14, CE15 and CE16 were tested by a group of 3 panelists. The results of the tests are given in Table 27.

**Table 27**

| Samples | Freshness index | Cleanliness index | Pleasant feeling index | Perm solution malodor | Evaluation |
|---|---|---|---|---|---|
| CE14 | OOE | OOE | OOE | 5.0 | Very strong perm odor |
| CE15 | OOE | OOE | OOE | 0.7 | Only a weak cold perm odor. Strong deodorizing effect observed. |
| CE16 | 18 | 23 | 21 | 3.0 | Initially fragrance odor, then cold perm odor felt. No significant cold perm deodorizing effect perceived. |
| E5 | 68 | 76 | 81 | 0 | No cold perm odor; initial fragrance retained. |

According to the results, unpleasant cold perm odor is eliminated by use of the deodorizing composition of the invention. Use of sample ESM-5 (comprising polyphenolic compounds and enzymes) alone already reduces cold perm odor. However, foul-smelling still remains. Use of a greenery- and floral-type fragrance alone shows no meaningful deodorizing effect, and initial green floral fragrance partly disappears.

### Embodiment 6 (E6)

A mouth rinse composition of the invention was tested for its deodorizing effect. 4 g of garlic was extracted with 1 liter of water. 10 ml of garlic extracts were mixed with 1 ml of mouth rinse, and the whole solution was stirred for 15 minutes at 25°C, and subjected to evaluation tests.

The mouth rinse product was prepared as follows. A phenol substrate was obtained by grinding dried green tea leaves and extracting ground tea leaves with hot water at 85 to 95°C for 2 hours. The tea extract was filtrated and the filtrate was washed three times with hexane. The water layer was condensed and dried to yield the phenol substrate. The latter contained approximately 20% by weight of polyphenolic compounds.

Enzymes were obtained from prematurely harvested pears. The latter were cut into slices, and the slices were freeze-dried and ground to obtain the enzyme powder. Specific enzyme activity was approximately 60 units/mg.

The substrate and the enzymes were mixed in a proportion of 1 . 1 (w/w), to obtain a sample ESM-6.

The mouth rinse product of the invention contained 0.5 wt % of peppermint-type flavor and 2 wt % of ESM-6.

The composition of an example of mouth rinse product of the invention is given in Table 28.

**Table 28**

| Materials | Weight % |
|---|---|
| Ethyl alcohol | 10.0 |
| Polyoxyethylene hydrogenated castor oil | 2.0 |
| Peppermint-type flavor | 0.5 |
| Saccarin Na | 0.02 |
| Glycerin | 10.0 |
| Na benzoate | 0.05 |
| FD & C color | Appropriately |
| ESM-6 | 2.0 |
| Purified water | Balance |
| Total | 100.0 |

### Comparative Example 17 (CE17)

A mouth rinse test solution was prepared without ESM-6 and flavor, and the solution was treated as described for E6.

### Comparative Example 18 (CE18)

A mouth rinse test solution was prepared with 2 wt % of ESM-6, but without flavor, and the solution was treated as described for E6.

### Comparative Example 19 (CE19)

A mouth rinse test solution was prepared with 0.5 wt % of peppermint-type flavor, but without ESM, and the solution was treated as described for E6.

The deodorizing effects of samples E6, CE17, CE18 and CE19 were tested and evaluated by a group of 3 panelists; The results of the tests are given in Table 29.

**Table 29**

| Samples | Freshness index | Cleanliness index | Pleasant feeling index | Malodor | Evaluation |
|---|---|---|---|---|---|
| CE17 | OOE | OOE | OOE | 5.0 | Very strong garlic odor. |
| CE18 | OOE | OOE | OOE | 0.6 | Only a weak garlic odor. Strong deodorizing effect observed. |
| CE19 | 26 | 21 | 20 | 2.4 | Initially mint flavor, then garlic odor felt. No significant garlic odor removal observed. |
| E6 | 78 | 78 | 76 | 0 | No garlic odor; initial peppermint flavor retained. |

According to the above results, unpleasant garlic odor is removed by the mouth rinse product of the invention. Use of sample ESM-6 (comprising polyphenolic compounds and enzymes) alone already reduces garlic odor, but foul-smelling still lingers. Use of a peppermint-type flavor alone does not remove garlic odor, and initial peppermint-type flavor partially disappears.

### Embodiment 7 (E7)

A deodorant composition of the invention was used in a toothpaste, and the mouth odor removal effect was evaluated.

The mouth of a subject (test person) was rinsed with water.

10 ml of 50 ppm methyl mercaptan solution were held in the mouth for one minute, and removed.

Exhaled air was collected in a 5 liter plastic bag.

The exhaled air was subjected to a group of 4 panelists for evaluation tests.

A phenol substrate was obtained by grinding dried peppermint leaves, and extracting the ground leaves with hot water for 2 hours at 85 to 95°C. The peppermint extract was filtered and the filtrate was washed three times with hexane. The water layer was condensed and dried to yield the phenol substrate. The latter contained approximately 20% by weight of polyphenolic compounds.

Enzymes were obtained from prematurely harvested pears. The latter were cut into slices, and the slices were freeze-dried and ground to obtain a enzyme powder. The specific enzyme activity was approximately 60 units/mg.

The substrate and the enzymes were mixed in a proportion of 1 : 1 (w/w), to obtain a sample ESM-7.

The toothpaste of the invention contained 1 wt % of mint-type flavor and 0.05 wt % of ESM-7.

The composition of an example of toothpaste of the invention is given in Table 29.

**Table 29**

| Materials | Weight % |
|---|---|
| Dicalcium phosphate | 10.0 |
| Sodium lauryl sulfate | 2.0 |
| Sodium carboxymethylcellulose | 0.5 |
| Sodium saccharin | 0.02 |
| Sodium benzoate | 10.0 |
| Glycerine | Balance |
| Mint-type flavor | 1.0 |
| ESM-7 | 0.05 |
| Total | 100.0 |

### Comparative Example 20 (CE20)

An exhaled air was collected without brushing the teeth, and the collected air was treated as described for E7.

### Comparative Example 21 (CE21)

After brushing teeth with toothpaste containing no ESM-7 nor mint-type flavor, an exhaled air was collected for evaluation tests.

### Comparative Example 22 (CE22)

After brushing teeth with toothpaste containing 0.05 wt % of ESM-7, an exhaled air was collected for evaluation tests.

### Comparative Example 23 (CE23)

After brushing teeth with toothpaste including 1 wt % of mint-type flavor, an exhaled air was collected for evaluation tests.

The results of the tests are given in Table 30.

**Table 30**

| Samples | Freshness index | Cleanliness index | Pleasant feeling index | Malodor | Evaluation |
|---|---|---|---|---|---|
| CE20 | OOE | OOE | OOE | 5.0 | Very strong methyl mercaptan odor. |
| CE21 | OOE | OOE | OOE | 3.3 | Very strong methyl mercaptan odor. |
| CE22 | OOE | OOE | OOE | 0.5 | Only a weak methyl mercaptan odor; strong deodorizing effect. |
| CE23 | 36 | 33 | 47 | 2.0 | Initially mint-type flavor, then methyl mercaptan odor. No perceptible deodorizing effect |
| E-7 | 80 | 88 | 79 | 0 | No methyl mercaptan odor; initial mint-type flavor retained. |

According to the above the results, unpleasant methyl mercaptan odor is obviated by use of the deodorizing composition of the invention. Use of ESM-7 (polyphenolic compounds plus enzymes) already reduces the methyl mercaptan odor, but this odor still remains. Use of a mint-type flavor alone does neutralize the methyl mercaptan odor. However, initial mint-type flavor partly disappears.

### Embodiment 8 (E8)

A tablet or candy containing a deodorant composition of the invention was tested for its deodorizing effect. The experiments were performed according to the following steps.

The mouth of a subject (test person) was rinsed with water.

10 ml of 50 ppm methyl mercaptan solution were held in the mouth for one minute, and removed.

Exhaled air was collected in a 5 liter plastic bag.

The subject licked the tablet for 10 minutes.

Exhaled air was collected in a 5 liter plastic bag.

The exhaled air in each plastic bag was subjected to a group of 4 panelists for evaluation tests.

A deodorizing tablet was prepared as follows.

A phenolic substrate used is an apple extract (product of NIKKA WHISKY KABUSHIKI KAISHA). The substrate contained about 75 wt % by weight of polyphenolic compounds.

Enzymes were obtained from potatoes (strain "Baron") by cutting the potatoes into slices, and freeze-drying and grinding the slices to obtain an enzyme powder. The specific activity of the powdered enzymes was about 30 units/mg.

The substrate and the enzymes were mixed in a proportion of 1 : 1 (w/w), to yield a sample ESM-8.

A tablet or candy containing 0.8 wt % by weight of mint-type flavor and 2.0 wt % by weight of ESM-8 was licked, and exhaled air was collected and tested as mentioned above.

The composition of an example of tablet is given in Table 31.

**Table 31**

| Materials | Weight % |
|---|---|
| Starches | 96.7 |
| Mint-type powdery flavor | 0.8 |
| Sucrose fatty acid ester | 0.5 |
| ESM-8 | 2.0 |
| Total | 100.0 |

### Comparative Example 24 (CE24)

A tablet containing no ESM-8 nor mint-type flavor was licked, and exhaled air was collected for evaluation tests.

### Comparative Example 25 (CE25)

A tablet containing ESM-8 was licked, and exhaled air was collected for evaluation tests.

### Comparative Example 26 (CE26)

A tablet containing a mint-type flavor was licked, and exhaled air was collected for evaluation tests.

The samples E8, CE24, CE25 and CE26 were subjected to a group of 4 panelists for quality evaluation. The results of their evaluations are given in Table 32 below.

**Table 32**

| Samples | Freshness index | Cleanliness index | Pleasant feeling index | Malodor | Evaluation |
|---|---|---|---|---|---|
| CE24 | OOE | OOE | OOE | 4.5 | Very strong methyl mercaptan odor. |
| CE25 | OOE | OOE | OOE | 0.5 | Only a weak methyl mercaptan odor. Strong deodorizing effect observed.. |
| CE26 | 33 | 38 | 32 | 2.8 | Initial mint-type flavor, then methyl mercaptan odor felt. No meaningful methyl mercaptan deodorizing effect perceived. |
| E8 | 80 | 88 | 74 | 0 | No methyl mercaptan odor. Initial mint-type flavor retained. |

According to the above results, unpleasant methyl mercaptan odor is eliminated by use of a deodorant composition of the invention. Use of sample ESM-8 (polyphenolic compounds + enzymes) alone already reduces methyl mercaptan odor, but this odor still lingers. Use of a mint-type flavor alone does neutralize methyl mercaptan odor, but initial mint-type flavor partially disappears.

### Embodiment 9 (E9)

A chewing gum containing a deodorant composition of the invention was prepared, and tested according to the following process.

The mouth of a subject (test person) was rinsed with water.

10 ml of 50 ppm methyl mercaptan solution were held in the mouth for one minute, and removed.

Exhaled air was collected in a 5 liter plastic bag.

The subject chewed the gum for 10 minutes.

Exhaled air was collected in a 5 liter plastic bag.

The exhaled air in each plastic bag was subjected to a group of 5 panelists for evaluation tests.

A deodorizing chewing gum was prepared as follows.

A phenolic substrate was obtained by extracting dried olive leaves with methanol under reflux at 60°C for 3 hours. The extracts were condensed to dryness, and dissolved in water. The water phase was filtered, and the filtrate was washed three times with hexane. The water phase was condensed and dried to yield the phenolic substrate. The substrate contained about 20 % by weight of polyphenolic compounds.

Enzymes were obtained from eggplants by cutting the eggplants into slices, and freeze-drying and grinding the slices, to obtain an enzyme powder. The specific activity of the powdered enzymes was about 30 units/mg.

The substrate and the enzymes were mixed in a proportion of 1 1 (w/w), to yield a sample ESM-9.

A gum containing 1.0 % by weight of lemon-type flavor and 1.0 % by weight of ESM-9 was chewed, and exhaled air was collected and tested as mentioned above.

The composition of an example of chewing gum is given in Table 33.

**Table 33**

| Materials | Weight % |
|---|---|
| Gum base | 21.0 |
| Sugar powder | 63.9 |
| Corn starch | 12.5 |
| Lemon-type flavor | 1.0 |
| Acidifier | 0.6 |
| ESM-9 | 1.0 |
| Total | 100.0 |

### Comparative Example 27 (CE27)

A gum containing no ESM-9 nor lemon-type flavor was chewed, and exhaled air was collected for evaluation tests.

### Comparative Example 28 (CE28)

A gum containing ESM-9 was chewed, and exhaled air was collected for evaluation tests.

### Comparative Example 29 (CE29)

A gum containing a lemon-type flavor was chewed, and exhaled air was collected for evaluation tests.

The samples E9, CE27, CE28 and CE29 were subjected to a group of 5 panelists for evaluation tests. The results of the tests are given in Table 34.

**Table 34**

| Samples | Freshness index | Cleanliness index | Pleasant feeling index | Malodor | Evaluation |
|---|---|---|---|---|---|
| CE27 | OOE | OOE | OOE | 4.2 | Very strong methyl mercaptan odor. |
| CE28 | OOE | OOE | OOE | 0.4 | Only a weak methyl mercaptan odor. Strong deodorizing effect observed. |
| CE29 | 33 | 28 | 30 | 3.2 | Initial lemon-type flavor, then methyl mercaptan odor. No meaningful deodorizing effect perceived. |
| E9 | 93 | 80 | 95 | 0 | No methyl mercaptan odor. Initial fresh lemon-type flavor retained. |

According to the above results, unpleasant methyl mercaptan odor is removed by the deodorizing composition of the invention. Use of sample ESM-9 (comprising polyphenolic compounds and enzymes) alone can reduce methyl mercaptan odor. However, foul-smelling still remains. Use of a lemon-type flavor does neutralize methyl mercaptan odor, but initial lemon-type flavor partially disappears.

### Embodiment 10 (E10)

A deodorant composition of the invention was applied to a food containing natto, which produces a strong odor. The food composition was prepared as follows.

A phenolic substrate was obtained from commercially available grape seed extracts (products of KIKKOMAN KABUSHIKI KAISHA). The extracts contained about 26 % by weight of polyphenolic compounds.

Enzymes were obtained by cutting yam into pieces, and freeze-drying and grinding the pieces, to yield an enzyme powder. The specific enzyme activity of the powder was about 30 units/mg.

The substrate and the enzyme powder were mixed in a proportion of 1 : 1 (w/w) to obtain a sample ESM-10.

50 g of natto were supplied with 0.05 g of ESM-10 and perilla-type flavor, and the whole mixture was blended at room temperature for 2 minutes. The resulting mixture was subjected to a group of 5 panelists for evaluation tests.

### Comparative Example 30 (CE30)

The sample contained no ESM-10 nor perilla-type flavor.

### Comparative Example 31 (CE31)

The sample contained ESM-10 but no flavor.

### Comparative Example 32 (CE32)

The sample contained perilla-type flavor, but no ESM-10.

The results of the evaluation tests are given in Table 35.

**Table 35**

| Samples | Freshness index | Cleanliness index | Pleasant feeling index | Malodor | Evaluation |
|---|---|---|---|---|---|
| CE30 | 00E | 00E | 00E | 5.1 | Strong amine odor |
| CE31 | 00E | 00E | 00E | 1.0 | No natto odor, but some pyrazine odor: strong natto deodorizing effect |
| CE32 | 33 | 28 | 30 | 3.2 | A weak but perceptible natto odor |
| E10 | 93 | 80 | 95 | 0 | No natto odor; initial perilla-type flavor retained. |

According to the above results, unpleasant natto odor is efficiently obviated by the inventive deodorant composition. When only the sample ESM-10 (e.g. polyphenolic compounds and enzymes) is used, pyrazine odor, instead of natto odor, is felt. When only the perilla-type flavor is used, natto odor cannot be removed completely, and the initial perilla flavor loses some effect.

### Embodiment 11 (E11)

In the present embodiment, some flavors containing sulfur atoms were studied, and their effect on the human olfactory sense was evaluated.

10 ml of water solution containing 100 ppm of methyl mercaptan (sodium salt) were prepared in a 50 ml vial bottle. 50 µl of coffee flavor (0.5 wt %) and 15 mg of ESM-1 were further added into the vial bottle, and the whole solution was stirred for 15 minutes at 25°C. The coffee flavor contained furfuryl mercaptan as essential perfumery compound. The composition of an example of coffee flavor is given in Table 36.

**Table 36**

| Flavor components | Wt % |
|---|---|
| Furfuryl mercaptan | 0.2 |
| Franeol | 0.3 |
| Cyclotene | 0.5 |
| Diacetyl | 0.6 |
| Furfural | 0.1 |
| Guaiacol | 0.5 |
| Isovaleric acid | 0.1 |
| Vanillin | 0.1 |
| Coffee extract | 50.0 |
| Water | 21.3 |
| Ethyl alcohol (95 %) | 26.3 |
| Total | 100.0 |

The coffee flavor was subjected to a group of 5 panelists for evaluation tests. The results of the tests are given in Table 37.

**Table 37**

| Flavor | Freshness index | Cleanliness index | Pleasant feeling index |
|---|---|---|---|
| Coffee flavor | 71 | 75 | 75 |

The effects of the flavors other than the coffee flavor, with respect to methyl mercaptan, were studied as described herebelow.

In the present study, A phenolic substrate was prepared according to the method described for Embodiment 1 (E1) using a grinder having a 5 mm mesh, to obtain a coffee extract. The extract contained about 30 % of polyphenolic compounds.

Likewise, enzymes were prepared according to the method described for E1. The obtained burdock powder had a specific enzyme activity of 200 units/mg.

The coffee extract and the burdock powder were mixed in a proportion of 1 : 1 (w/w), to yield a sample ESM-1'.

The general flavor levels were evaluated by a group of 5 panelists, on the basis of "freshness", "cleanliness" and "pleasant feeling". The results of their evaluation were given as points ranging from 0 to 100.

By contrast, the malodor levels were classified into 5 categories:
0: no smelling;
1: very weak smelling;
2: weak smelling;
3: smelling;
4: strong smelling;
5: very strong smelling.

### Comparative Example 1' (CE1')

10 ml of methy mercaptan (sodium salt) and 10 µl of water were placed into a vial bottle, and the mixture was reacted as described for E1'.

### Comparative Example 2' (CE2')

10 ml of methy mercaptan (sodium salt) and 10 µl of water were placed into a vial bottle, and the mixture was reacted as described for E1'.

### Comparative Example 3' (CE3')

10 ml of methy mercaptan (sodium salt) and 50 µl of coffee flavor were placed into a vial bottle, and the mixture was reacted as described for E1'.

### Comparative Example 4' (CE4')

10 ml of methy mercaptan (sodium salt) and 50 µl of peppermint-type flavor were placed into a vial bottle, and the mixture was reacted as described for E1'.

### Comparative Example 5' (CE5')

10 ml of methy mercaptan (sodium salt), 15 mg of ESM-1' and 50 µl of peppermint-type flavor were placed into a vial bottle, and the mixture was reacted as described for E1'.

The results of evaluation tests are given in Table 38.

**Table 38**

| Samples | Freshness index | Cleanliness index | Pleasant feeling index | Malodor index | Evaluation |
|---|---|---|---|---|---|
| CE1' | 00E | 00E | 00E | 5.0 | Strong methyl mercaptan odor |
| CE2' | 00E | 00E | 00E | 0.3 | Slight sulfur odor. Weak odor originating from ESM-1' |
| CE3' | 26 | 21 | 20 | 3.2 | Initially coffee flavor, then strong methyl mercaptan odor |
| E11 | 10 | 10 | 5 | 3.6 | Disintegrated weak coffee flavor; methyl mercaptan odor identifiable |
| CE4' | 26 | 21 | 20 | 2.4 | Initially peppermint flavor, then strong methyl mercaptan odor |
| CE5' | 78 | 78 | 76 | 0.0 | No methyl mercaptan odor; initial peppermint flavor retained |

## Claims

1. A deodorant composition comprising at least one phenolic compound and at least one enzyme capable of oxidizing the at least one phenolic compound into a compound having a quinone structure, **characterized in that**
said deodorant composition further comprises at least one of flavor and fragrance that comprises at least one compound selected from the group consisting of one or several volatile compounds having a molecular weight lower than 500 Dalton; an essential oil; and plant, vegetable or fruit extracts, said deodorant composition comprising said at least one of flavor and fragrance in an amount sufficient to remove the last remaining trace of odours which cannot be inactivated by said at least one phenolic compound and at least one enzyme;
wherein said at least one of flavor and fragrance is citrus-type fragrance, mint-type fragrance, musk- and wood-type fragrance, fruit- and floral-type fragrance, greenery- and floral-type fragrance, peppermint-type flavour, mint-type flavour, lemon-type flavour or perilla-type flavor

2. A deodorant composition according to claim 1, wherein the amount of said at least one of flavour and fragrance compound to be mixed is at least 0.1% by weight, with respect to the amount of said deodorant composition.

3. A deodorant composition according to claim 1, wherein the amount of said at least one of flavour and fragrance compound to be mixed is at least 1% by weight, with respect to the amount of said deodorant composition.

4. A deodorant composition according to claim 3, wherein said one or several volatile compounds having a molecular weight lower than 500 Dalton contain no sulfur atom nor nitrogen atom.

5. A deodorant composition according to claim 3, wherein, when said at least one of flavor and fragrance comprises several compounds and at least one of said several compounds includes a sulfur atom or nitrogen atom, said sulfur- or nitrogen-including compound does not constitute an essential perfumery component in said at least one of fragrance and flavor.

6. A deodorant composition according to any one of claims 1 to 5, wherein said enzyme comprises at least one enzyme selected from the group consisting of polyphenol oxidase, cathecol oxidase, monophenol oxidase, peroxidase and a plant extract containing an oxidase.

7. A deodorant composition according to claim 6, wherein said polyphenol oxidase comprises a least one enzyme selected from the group consisting of laccase, urushiol oxidase and phenolase.

8. A deodorant composition according to claim 6, wherein said monophenol oxydase comprises tyrosinase.

9. A product or article comprising the deodorant composition containing said phenolic compound(s), said enzyme(s), and said flavour and/or fragrance defined in any one of claims 1 to 8, as a combined preparation for simultaneous, separate or sequential use.

10. A product or article according to claim 9, wherein said product or article comprises a detergent, an antiperspirant, a shampoo, a mouth rinse, toothpaste, tablet, candy, a chewing gum, or a food.

11. A spray product containing the deodorant composition defined in any one of claims 1 to 8.

12. A method of removing and/or masking malodors comprising the steps of:
mixing at least one phenolic compound, at least one enzyme capable of oxidizing said phenolic compound(s) and at least one of fragrance and flavour, whereby there is provided a deodorant composition defined in any one of claims 1 to 8; and
applying said deodorant composition to a malodorous object, human or mammal.

13. A method of removing and/or masking malodors according to claim 12, wherein said mixing step comprises an appropriate carrier, whereby there is provided a carrier-bearing composition including a deodorant composition defined in any one of claims 1 to 8.

14. A method of removing and/or masking malodors according to claim 12, wherein said mixing step comprises an appropriate carrier, whereby there is provided a deodorant product or article defined in claim 9 or 10.

## Patentansprüche

1. Deodorantzusammensetzung, umfassend zumindest eine Phenolverbindung und zumindest ein Enzym, das in der Lage ist, die zumindest eine Phenolver-bindung zu einer Verbindung zu oxidieren, die eine Chinonstruktur aufweist, **dadurch gekennzeichnet, dass**
die Deodorantzusammensetzung ferner umfasst: zumindest eines von Duftstoff und Parfüm, der bzw. das zumindest eine Verbindung umfasst, die ausgewählt ist aus der Gruppe bestehend aus einer oder mehreren flüchtigen Verbindung mit einem Molekulargewicht niedriger als 500 Dalton; ätherisches Öl; und Pflanzen-, Gemüse- oder Früchteextrakte, wobei die Deodorantzusammensetzung das zumindest eine von Duftstoff und Parfüm in einer Menge umfasst, die ausreichend ist, um die letzte verbleibende Spur von Gerüchen zu entfernen, die nicht von der zumindest einen Phenolverbindung und dem zumindest einen Enzym inaktiviert werden können;
wobei das zumindest eine von Duftstoff und Parfüm, Parfüm vom Zitrustyp, Parfüm vom Minztyp, Parfüm vom Moschus- und Holztyp, Parfüm vom Früchte- und Blumentyp, Parfüm vom Grünpflanzen- und Blumentyp, Parfüm vom Pfefferminztyp, Parfüm vom Minztyp, Parfüm vom Zitronentyp oder Parfüm vom Perillatyp ist.

2. Deodorantzusammensetzung nach Anspruch 1, wobei die Menge der zumindest einen von Duftstoff- und Parfümverbindung, die zu mischen ist, zumindest 0,1 Gewichtsprozent bezogen auf die Menge der Deodorantzusammensetzung beträgt.

3. Deodorantzusammensetzung nach Anspruch 1, wobei die Menge der zumindest einen von Duftstoff- und Parfümverbindung, die zu mischen ist, zumindest 1 Gewichtsprozent bezogen auf die Menge der Deodorantzusammensetzung beträgt.

4. Deodorantzusammensetzung nach Anspruch 3, wobei die eine oder die mehreren flüchtigen Verbindungen, die ein Molekulargewicht niedriger als 500 Dalton aufweisen, weder ein Schwefelatom noch ein Stickstoffatom enthalten.

5. Deodorantzusammensetzung nach Anspruch 3, wobei, wenn das zumindest eine von Duftstoff und Parfüm mehrere Verbindungen umfasst und zumindest eine der mehreren Verbindungen ein Schwefelatom oder Stickstoffatom enthält, die schwefel- oder stickstoffenthaltende Verbindung keine essentielle Parfümeriekomponente in dem zumindest einen von Duftstoff und Parfüm darstellt.

6. Deodorantzusammensetzung nach einem der Ansprüche 1 bis 5, wobei das Enzym zumindest ein Enzym umfasst, das ausgewählt ist aus der Gruppe bestehend aus Polyphenoloxidase, Catecholoxidase, Monophenoloxidase, Peroxidase und einem Pflanzenextrakt, der eine Oxidase enthält.

7. Deodorantzusammensetzung nach Anspruch 6, wobei die Polyphenoloxidase zumindest ein Enzym fasst, das ausgewählt ist aus der Gruppe bestehend aus Laccase, Urushioloxidase und Phenolase.

8. Deodorantzusammensetzung nach Anspruch 6, wobei die Monophenoloxidase Tyrosinase umfasst.

9. Produkt oder Artikel, umfassend die Deodorantzusammensetzung, welche die Phenolverbindung(en), das/die Enzym(e), und den Duftstoff und/oder Parfüm, die in einem der Ansprüche 1 bis 8 definiert sind, als eine kombinierte Zubereitung zur gleichzeitigen, separaten oder sequentiellen Verwendung enthält.

10. Produkt oder Artikel nach Anspruch 9, wobei das Produkt oder der Artikel ein Reinigungsmittel, ein Antitranspirant, ein Shampoo, eine Mundspülung, Zahnpaste, Tablette, Süßigkeit, ein Kaugummi oder ein Lebensmittel umfasst.

11. Sprayprodukt, enthaltend die Deodorantzusammensetzung, die in einem der Ansprüche 1 bis 8 definiert ist.

12. Verfahren zum Entfernen und/oder Verdecken von schlechten Gerüchen, umfassend die Schritte:
Mischen zumindest einer Phenolverbindung, zumindest eines Enzyms, das in der Lage ist, die Phenolverbindung(en) zu oxidieren, und zumindest eines von Parfüm und Duftstoff, wodurch eine Deodorantzusammensetzung bereitgestellt wird, wie sie in einem der Ansprüche 1 bis 8 definiert ist; und
Anwenden der Deodorantzusammensetzung an einem übelriechenden Objekt, Mensch oder Säugetier.

13. Verfahren zum Entfernen und/oder Verdecken von schlechten Gerüchen nach Anspruch 12, wobei der Mischschritt einen geeigneten Träger umfasst, wodurch eine trägertragende Zusammensetzung bereitgestellt wird, die eine Deodorantzusammensetzung enthält, wie sie in einem der Ansprüche 1 bis 8 definiert ist.

14. Verfahren zum Entfernen und/oder Verdecken von schlechten Gerüchen nach Anspruch 12, wobei der Mischschritt einen geeigneten Träger umfasst, wodurch ein Deodorantprodukt oder -artikel bereitgestellt wird, wie er in Anspruch 9 oder 10 definiert ist.

## Revendications

1. Composition désodorisante comprenant au moins un composé phénolique et au moins une enzyme capable d'oxyder le ou les composés phénoliques en un composé présentant une structure de quinone, **caractérisée en ce que**
ladite composition désodorisante comprend, en outre, au moins l'un d'une saveur et d'un parfum qui comprend au moins un composé sélectionné à partir du groupe constitué par un ou plusieurs composés volatils présentant un poids moléculaire inférieur à 500 Dalton ; une huile essentielle, et des extraits de plante, de légume ou de fruit, ladite composition désodorisante comprenant ledit au moins l'un d'une saveur et d'un parfum en une quantité suffisante afin d'éliminer les dernières traces résiduelles d'odeurs qui ne peuvent pas être inactivées par le ou lesdits composés phénoliques et la ou lesdites enzymes ;
dans laquelle ledit au moins l'un d'une saveur et d'un parfum est un parfum du type citron, un parfum du type menthe, un parfum du type musc et bois, un parfum du type fruit et floral, un parfum du type verdure et floral, une saveur du type menthe poivrée, une saveur du type menthe, une saveur du type citron, ou une saveur du type pérille.

2. Composition désodorisante selon la revendication 1, dans laquelle la quantité dudit composé d'au moins l'un d'une saveur et d'un parfum à mélanger est supérieure ou égale à 0,1% en poids, par rapport à la quantité de ladite composition désodorisante.

3. Composition désodorisante selon la revendication 1, dans laquelle la quantité dudit composé d'au moins l'un d'une saveur et d'un parfum à mélanger est supérieure ou égale à 1% en poids, par rapport à la quantité de ladite composition désodorisante.

4. Composition désodorisante selon la revendication 3, dans laquelle le ou lesdits composés volatils présentant un poids moléculaire inférieur à 500 Dalton ne contiennent pas d'atome de soufre ni d'atome d'azote.

5. Composition désodorisante selon la revendication 3, dans laquelle, ledit au moins l'un d'une saveur et d'un parfum comprend plusieurs composés et au moins l'un desdits plusieurs composés comporte un atome de soufre ou un atome d'azote, ledit composé comportant du soufre ou de l'azote ne constitue pas un composant essentiel de parfumerie dans ledit au moins l'un d'une saveur et d'un parfum.

6. Composition désodorisante selon l'une quelconque des revendications 1 à 5, dans laquelle
ladite enzyme comprend au moins une enzyme sélectionnée à partir du groupe constitué par la polyphénol oxydase, la cathécol oxydase, la monophénol oxydase et la peroxydase et un extrait de plante contenant de l'oxydase.

7. Composition désodorisante selon la revendication 6, dans laquelle ladite polyphénol oxydase comprend au moins une enzyme sélectionnée à partir du groupe constitué par la laccase, l'urushiol oxydase et la phénolase.

8. Composition désodorisante selon la revendication 6, dans laquelle ladite monophénol oxydase comprend la tyrosinase.

9. Produit ou article comprenant la composition désodorisante contenant le ou lesdits composés phénoliques, la ou lesdites enzymes, ledit parfum et/ou saveur selon l'une quelconque des revendications 1 à 8, sous la forme d'une préparation mélangée pour une utilisation simultanée, séparée ou séquentielle.

10. Produit ou article selon la revendication 9, dans lequel ledit produit ou article comprend un détergent, un anti-transpirant, un shampooing, un bain de bouche, un dentifrice, un comprimé, un bonbon, un chewing-gum ou un aliment.

11. Produit à pulvériser contenant la composition désodorisante selon l'une quelconque des revendications 1 à 8.

12. Procédé d'élimination et/ou de masquage de mauvaises odeurs comprenant les étapes de :
mélange d'au moins un composé phénolique, d'au moins une enzyme capable d'oxyder le ou lesdits composés phénoliques et d'au moins l'un d'un parfum et d'une saveur, de telle sorte qu'il est formé une composition désodorisante selon l'une quelconque des revendications 1 à 8 ; et
application de ladite composition désodorisante sur un objet malodorant, humain ou mammifère.

13. Procédé d'élimination et/ou de masquage de mauvaises odeurs selon la revendication 12, dans lequel ladite étape de mélange comprend un excipient approprié, de telle sorte qu'il est formé une composition contenant un excipient comportant une composition désodorisante selon l'une quelconque des revendications 1 à 8.

14. Procédé d'élimination et/ou de masquage de mauvaises odeurs selon la revendication 12, dans lequel ladite étape de mélange comprend un excipient approprié, de telle sorte qu'il est formé un produit ou article désodorisant selon la revendication 9 ou 10.
